Europäisches Patentamt

European Patent Office

Office européen des brevets

(19)

(11) Veröffentlichungsnummer: **0 283 573 B1**

(12) **EUROPÄISCHE PATENTSCHRIFT**

(45) Veröffentlichungstag der Patentschrift: **04.03.92**

(51) Int. Cl.5: **A61K 9/18**, A61K 47/00

(21) Anmeldenummer: **87118320.8**

(22) Anmeldetag: **10.12.87**

(54) **Wasserfreie Applikationsform niedermolekularer Alkalihuminate.**

(30) Priorität: **21.03.87 DE 3709353**

(43) Veröffentlichungstag der Anmeldung:
**28.09.88 Patentblatt 88/39**

(45) Bekanntmachung des Hinweises auf die
Patenterteilung:
**04.03.92 Patentblatt 92/10**

(84) Benannte Vertragsstaaten:
**AT BE CH DE ES FR GB GR IT LI LU NL SE**

(56) Entgegenhaltungen:
**DE-A- 2 331 097
DE-C- 712 697
DE-C- 888 444**

(73) Patentinhaber: **RÜTGERSWERKE AKTIENGE-
SELLSCHAFT**
**Mainzer Landstrasse 217**
**W-6000 Frankfurt am Main 1(DE)**

(72) Erfinder: **Seubert, Bernhard**
**Meisenweg 15**
**W-6803 Edingen-Neckarhausen 1(DE)**
Erfinder: **Beilharz, Helmut, Dr.**
**Steinachstr. 14**
**W-6905 Schriesheim(DE)**
Erfinder: **Fickert, Werner, Dr.**
**Stockacher Str. 14**
**W-6800 Mannheim 61(DE)**
Erfinder: **Jeromin, Günter, Dr.**
**Bergstr. 14**
**W-6900 Heidelberg(DE)**
Erfinder: **Spitaler, Ulrich, Dr.**
**Dürkheimer Hohl 2**
**W-6713 Freinsheim(DE)**

# Beschreibung

Die Erfindung betrifft eine neue Applikationsform niedermolekularer Alkalihuminate.

Gemäß früherer Anmeldungen (EP-A 0 281 678 und EP-A 0 281 679) lassen sich diese niedermolekularen Alkalihuminate als Heilmittel insbesondere bei der Wundheilung einsetzen.

Sie werden dabei meist als 1 bis 5 %ige wäßrige Lösung eingesetzt. Derartige Lösungen lassen sich zwar zu höheren Konzentrationen aufkonzentrieren, jedoch lassen sich auch mit den schonendsten Verfahren keine wasserfreien niedermolekularen Alkalihuminate herstellen, da diese dabei irreversibel zerstört werden.

Anderseits benötigt man für verschiedene Darreichungsformen, wie etwa wasserfreie Salben, Haftpasten oder Sprays den Wirkstoff wasserfrei.

Es bestand daher die Aufgabe, die als Wirkstoffe einzusetzenden niedermolekularen Alkalihuminate in einer wasserfreien Applikationsform zu erhalten, aus der sie bei Kontakt mit Wasser aber leicht freigesetzt werden können, ohne daß ihre Wirksamkeit gegenüber der der ursprünglichen wäßrigen Lösung verändert ist.

Die Lösung der Aufgabe erfolgt durch die Bereitstellung der wasserfreien Applikationsform gemäß der Ansprüche 1 und 2 und das Verfahren zu Ihrer Herstellung gemäß der Ansprüche 3 und 4. Die wasserfreie Applikationsform kann zur Herstellung von wasserfreien Salben, Pudern, Haftpasten oder Sprays verwendet werden.

Es wurde gefunden, daß sich niedermolekulare Alkalihuminate entwässern lassen, ohne sich chemich zu verändern, wenn sie im Gemisch mit wasserstoffbrückenbildendem anorganischem Trägermaterial vorliegen. Die dabei entstehenden Komplexe oder Addukte sind stabile, rieselfähige Produkte aus denen sich durch Zugabe von Wasser die niedermolekularen Alkalihuminate in ihrem ursprünglichen Aufbau und ihter ursprünglichen physiologischen Aktivität freisetzen lassen. Erfindungsgemäß einsetzbares Trägermaterial sind wasserstoffbrückenbildende anorganische Stoffe wie z.B. Aluminiumoxid, Titandioxid hochdisperses Siliciumdioxid oder Ton, insbesondere Montmorillonit oder Bentonit.

Es ist bekannt (Ziechmann, "Huminstoffe", Verlag Chemie 1980, 284 ff), daß Huminstoffe mit Tonen Komplexe bilden. Es ist aber von diesen Komplexen bekannt, daß die Huminstoffe, katalysiert durch das Tonmaterial, zu höhermolekularen Stoffen weiteroxidiert werden. Außerdem können die Huminstoffe aus diesen Ton-Komplexen mit Wasser nicht mehr freigesetzt werden, sondern nur noch durch Einwirkung von Alkalilauge bei erhöhter Temperatur, wobei ihre Struktur verändert wird.

Es ist daher überraschend, daß die (natürlichen und synthetischen) niedermolekularen Alkalihuminate mit einem mittlerem Molekulargewicht von 1000 mit einem Streubereich von 300 bis 1500 mit z.B. Tonmaterial Komplexe oder Addukte bilden, die einerseits so stabil sind, daß sie nicht der Autoxidation oder sonstigen Humifizierungsprozessen unterliegen und die aber andererseits wieder durch die Hydratationskräfte des Wassers bei Raumtemperatur gespalten werden können.

Es ist weiterhin überraschend, daß die labilen Huminatmoleküle, wenn sie an wasserstoffbrückenbildendes anorganisches Trägermaterial fixiert sind, sich entwässern lassen ohne zu weiteren Dehydrationsreaktionen zu neigen, ja, daß sie sich ohne Veränderungen zu erleiden bis auf Temperaturen von 110° C erhitzen lassen.

Niedermolekulare Alkalihuminate sind hygroskopische Substanzen, wenn sie weniger als 5 % Wasser enthalten. Es ist ein weiterer überraschender Vorteil, daß diese Eigenschaft verloren geht, wenn die Alkalihuminate wasserfrei an das anorganische Trägermaterial gebunden sind.

Zur Herstellung der wasserfreien Applikationsform wird die bei der Herstellung der niedermolekularen Alkalihuminate erhaltene 1 - 5 %ige wäßrige Lösung oder eine aufkonzentrierte Lösung mit dem 0,2 - 5fachen Volumen des anorganischen Trägermaterials angeteigt.

Die so erhaltene Mischung wird in an sich bekannter Weise entweder durch Vakuumbehandlung und oder durch Erhitzen auf Temperaturen bis zu 110° C getrocknet, wobei es zweckmäßig ist, die Masse während des Trocknungsvorganges zu rühren, um eine Verklumpung zu vermeiden.

Das dann erhaltene rieselfähige braune Pulver ist ohne weitere Schutzmaßnahmen lagerstabil kann alleine oder mit Hilfsstoffen als Puder oder in Sprays eingesetzt werden oder läßt sich leicht mit wasserfreien Salbengrundlagen wie Vaseline oder dickflüßigem Paraffin zu einer wasserfreien Paste verreiben.

## Patentansprüche

1. Wasserfreie Applikationsform niedermolekularer Alkalihuminate, **dadurch gekennzeichnet,** daß die wasserfreien, niedermolekularen Alkalihuminate mit einem mittleren Molekulargewicht von 1.000 und einem Streubereich von 300 bis 1.500 an Titandioxid, Aluminiumoxid, hochdisperses Siliciumdioxid oder Ton als Trägermaterial gebunden sind.

2. Wasserfreie Applikationsform nach Anspruch 1, **dadurch gekennzeichnet,** daß das anorganische Trägermaterial ein Montmorillonit oder ein Bentonit ist.

3. Verfahren zur Herstellung der wasserfreien Applikationsform nach den Ansprüchen 1 und 2, **dadurch gekennzeichnet,** daß die wäßrige Lösung niedermolekularer Alkalihuminate mit einem mittleren Molekulargewicht von 1.000 und einem Streubereich von 300 bis 1.500 mit dem anorganischen Trägermaterial vermischt und die erhaltene Mischung getrocknet wird.

4. Verfahren nach Anspruch 3, **dadurch gekennzeichnet,** daß die Trocknung durch Erhitzen der Mischung auf Temperaturen bis zu 110 ˚C erfolgt.

5. Verwendung der wasserfreien Applikationsform nach den Ansprüchen 1 und 2 zur Herstellung von wasserfreien Applikationsform nach den Ansprüchen 1 bis 3 zur Herstellung von wasserfreien Salben, Haftpasten, Pudern oder Sprays.

## Claims

1. An anhydrous administration form of low-molecular alkali huminates, characterized in that the anhydrous low-molecular alkali huminates with an average molecular weight of 1,000 and a scatter of from 300 to 1,500 are bonded to titanium dioxide, aluminium oxide, highly dispersed silicon dioxide or clay as the carrier material.

2. An anhydrous administration form according to claim 1, characterized in that the inorganic carrier material is a montmorillonite or a bentonite.

3. A process for preparing the anhydrous administration form according to Claims 1 and 2, characterized in that the aqueous solution of low-molecular alkali huminates with an average molecular weight of 1,000 and a scatter of from 300 to 1,500 is mixed with the inorganic carrier material and the mixture obtained is dried.

4. A process according to Claim 3, characterized in that the drying is performed by heating the mixture to temperatures of up to 110˚ C.

5. Use of the anhydrous administration form according to Claims 1 and 2 for preparing [the] anhydrous administration form according to Claims 1 to 3 [*sic*] for preparing anhydrous ointments, adhesive pastes, powders or sprays.

## Revendications

1. Forme d'application anhydre d'humates alcalins de bas poids moléculaire, caractérisée en ce que les humates alcalins anhydres de bas poids moléculaire d'un poids moléculaire moyen de 1000 avec une plage de dispersion de 300 à 1500 sont liés à du dioxyde de titane, de l'alumine, de la silice fortement dispersée ou de l'argile en tant que support.

2. Forme d'application anhydre selon la revendication 1, caractérisée en ce que le support inorganique est une montmorillonite ou une bentonite.

3. Procédé pour la préparation de la forme d'application anhydre selon les revendications 1 et 2, caractérisé en ce que la solution aqueuse d'humates alcalins de bas poids moléculaire d'un poids moléculaire moyen de 1000 avec une plage de dispersion de 300 à 1500 est mélangée avec le support inorganique et que le mélange obtenu est séché.

4. Procédé selon la revendication 3, caractérisé en ce que le séchage a lieu par chauffage du mélange à des températures jusqu'à 110˚ C.

5. Utilisation de la forme d'application anhydre selon les revendications 1 et 2 pour la préparation d'une forme d'application anhydre selon les revendications 1 à 3 destinée à la préparation de pommades, pâtes adhésives, poudres ou sprays anhydres.